Europäisches Patentamt

European Patent Office

Office européen des brevets

(19)

(11) Veröffentlichungsnummer: **0 264 821**
**B1**

(12)   EUROPÄISCHE PATENTSCHRIFT

(45) Veröffentlichungstag der Patentschrift: **09.01.91**

(51) Int. Cl.⁵: **A 61 F 9/02**

(21) Anmeldenummer: **87115059.5**

(22) Anmeldetag: **15.10.87**

(54)  Skibrille, Arbeitsschutz-Brille, Motorradhelm-Visier o. dgl.

(30) Priorität: **21.10.86 DE 3635703**

(43) Veröffentlichungstag der Anmeldung:
**27.04.88 Patentblatt 88/17**

(45) Bekanntmachung des Hinweises auf die
Patenterteilung:
**09.01.91 Patentblatt 91/02**

(84) Benannte Vertragsstaaten:
**AT CH DE FR IT LI**

(56) Entgegenhaltungen:
**DE-A-2 556 154**
**DE-A-3 210 960**
**DE-A-3 323 419**
**FR-A-2 316 913**
**FR-A-2 542 462**

(73) Patentinhaber: **UVEX WINTER OPTIK GMBH**
**Salzstrasse 18-22**
**D-8510 Fürth/Bay (DE)**

(72) Erfinder: **Wiedner, Klaus, Dipl.-Kaufm.**
**Coubertinstrasse 28**
**D-8510 Fürth/Bay (DE)**

(74) Vertreter: **Schneck, Herbert, Dipl.-Phys., Dr. et al**
**Rau & Schneck Patentanwälte Königstrasse 2**
**D-8500 Nürnberg 1 (DE)**

Courier Press, Leamington Spa, England.

EP 0 264 821 B1

## Beschreibung

Die Erfindung richtet sich auf eine Skibrille, Arbeitsschutzbrille, ein Motorradhelm-Visier oder dergleichen mit einer Scheibe umfassend zwei mittels eines Klebers verbundene transparente Kunststoffscheiben, wobei wenigstens die innere Scheibe eine Cellulosederivatscheibe ist, welche mit der zweiten Scheibe mittels eines Klebstoffes verklebt ist.

An derartige Brillen und deren Scheiben werden sehr unterschiedliche, teilweise miteinander konkurrierende Anforderungen gestellt. Insbesondere müssen die Brillen, z. b. bei Skibrillen, wenn diese um den Arm geschlungen getragen werden, verwindungsfähig sein, so daß die Scheiben eine relativ hohe Elastizität aufweisen müssen. Weiterhin muß zur Erzielung befriedigender optischer Eigenschaften die Transparenz der Sichtscheibe gut sein. Die Sichtscheibe soll an ihrer Aussenseite eine möglichst hohe Kratzfestigkeit aufweisen und an ihrer Innenseite im Tragezustand möglichst wenig beschlagen.

In der DE—OS—33 23 419 und der DE—OS—32 10 960 werden Skibrillen mit Sichtscheiben beschrieben, welche aus zwei Scheiben bestehen, welche längs ihres Randbereiches miteinander verklebt sind. Gemäß der DE—OS—33 23 419 ist vorgesehen, daß die zweite Scheibe eine sehr dünne Polycarbonat-Folie ist, welche auf Abstand zu der tragenden polycarbonat-Scheibe gehalten wird. Derartige Polycarbonat-Scheiben lassen sich jedoch nicht mit einer wirksamen Antibeschlag-Beschichtung versehen. Gemäß der DE—OS—32 10 960 die alle Merkmale des Oberbegriffs des Anspruch 1 aufweist sind die beiden Scheiben ebenfalls auf Abstand zueinander gehalten und über einen elastischen, gummiartigen Zwischenring miteinander verklebt.

Diese vorbekannten Lösungen sind herstellungstechnisch aufwendig und die fertigen Scheiben weisen gleichwohl noch unbefriedigende Gebrauchseigenschaften auf, da einerseits eine relativ dünne Folie der Gefahr von Beschädigungen unterliegt und andererseits zwei im Abstand miteinander verbundene eigensteife Scheiben eine relativ geringe Verwindungsfähigkeit aufweisen.

Es ist weiterhin bekannt, auf eine transparente Kunststoffscheibe auf chemischem Weg eine Beschichtung zur Erzielung einer Antibeschlagwirkung aufzubringen. Dieses herstellungstechnisch an sich sehr rationelle und im Ergebnis wirkungsvolle Verfahren läßt sich allerdings nur bei aus Zellulosederivaten hergestellten Scheiben wirkungsvoll realisieren. Diese Scheiben weisen aber wiederum den Nachteil auf, daß sie nicht so kratzfest sind, wie dies für viele Einsatzzwecke wünschenswert und notwendig ist, wobei sich die gewünschte Kratzfestigkeit auch nicht durch eine nachträgliche Behandlung mit vertretbarem Aufwand herstellen läßt. Demgegenüber kann bei Polycarbonat-Scheiben die Kratzfestigkeit durch Aufbringen einer entsprechenden Lackbeschichtung verbessert werden.

Hiervon ausgehend liegt der Erfindung die Aufgabe zugrunde, eine Brille oder ein Visier der eingangs genannten Art so auszugestalten, daß die Scheibe verwindungsfähig ist, daß sie an ihrer Außenseite eine gute Kratzfestigkeit aufweist und daß ein Beschlagen der Innenseite vermieden wird.

Diese Aufgabe wird erfindungsgemäß dadurch gelöst, daß die beiden Scheiben mittels eines transparenten Klebstoffes auf Silikonkautschuk-Basis flächig verklebt sind.

Durch eine derartige flächige Verklebung werden die günstigen Eigenschaften einer Verbundscheibe, d. h. insbesondere eine sehr hohe mechanische Festigkeit erreicht. Der verwendete Klebstoff auf Silikonkautschuk-Basis weist eine an sich bekannte, sehr gute Transparenz auf, so daß sich eine flächige Verklebung realisieren läßt, zeichnet sich darüber hinaus aber auch durch eine gewisse Elastizität und ein geringes Wärmeleitungsvermögen aus, so daß ähnlich wie bei im Abstand voneinander angeordneten Scheiben bereits aufgrund der Wärmeisolierung ein Beschlagen der Innenseite weitgehend verhindert wird. Darüber hinaus ermöglicht es die Verwendung einer Cellulosederivat-Scheibe, zusätzlich eine Antibeschlag-Beschichtung an der Innenseite der Scheibe anzubringen. Letztlich ist die erfindungsgemäße Verbundscheibe auch herstellungstechnisch besonders preisgünstig realisierbar, da sich der Scheibenverbund als solcher in Form flächiger Platten herstellen läßt und die einzelnen Scheiben aus diesen Platten ausgestanzt werden können, so daß kein individuelles Verkleben der beiden Teilscheiben bei der Herstellung erforderlich ist.

Vorzugsweise ist vorgesehen, daß der Klebstoff auf der Basis von Polydiacryl, Polydiarylbzw. Polyalkylarylsiloxanen mit reaktionsfähigen, insbesondere aciden Endgruppen hergestellt ist. Derartige Klebstoffe werden den spezifischen Anforderungen für die Verwendung z.B. bei Skibrillen in besonders vorteilhafter Weise gerecht, weil sie eine sehr hohe Transparenz, Eigenelastizität und ein niedriges Wärmeleitungsvermögen aufweisen.

Mit besonderem Vorteil ist vorgesehen, daß die zweite Scheibe eine Polycarbonatscheibe ist. Es ist dann möglich, diese Polycarbonatscheibe in an sich bekannter Weise mit sehr wirtschaftlichen Herstellungstechniken und mit hoher Wirksamkeit an ihrer Außenseite mit einer die Kratzfestigkeit erhöhenden Beschichtung zu versehen. Andererseits kann die Zellulosederivat-Scheibe ebenfalls an ihrer Außenseite mit einer Antibeschlag-Beschichtung, versehen werden, welche insbesondere durch Verseifen der Oberfläche der Zellulosederivat-Scheibe realisiert werden kann.

Eine derartige Verbundscheibe, welche durch eine flächig verklebte Polycarbonatscheibe mit einer Zellulosederivat-Scheibe gebildet ist und im Gebrauchszustand der Brille oder des Visiers an der Außenseite eine außerordentlich hohe Kratzfestigkeit und an der Innenseite eine hohe Beschlagfreiheit aufweist, wird den an sie gestellten, besonderen Eigenschaften in idealer Weise gerecht.

Weitere Merkmale, Vorteile und Einzelheiten der Erfindung ergeben sich aus der folgenden Beschreibung einer bevorzugten Ausführungsform anhand der Zeichnung. Dabei zeigen

Fig. 1 eine schematische, perspektivische Ansicht einer erfindungsgemäßen Skibrille und

Fig. 2 einen schematischen Schnitt durch die Scheibe einer erfindungsgemäßen Skibrille, Arbeitsschutz-Brille, eines Visiers od.dgl.

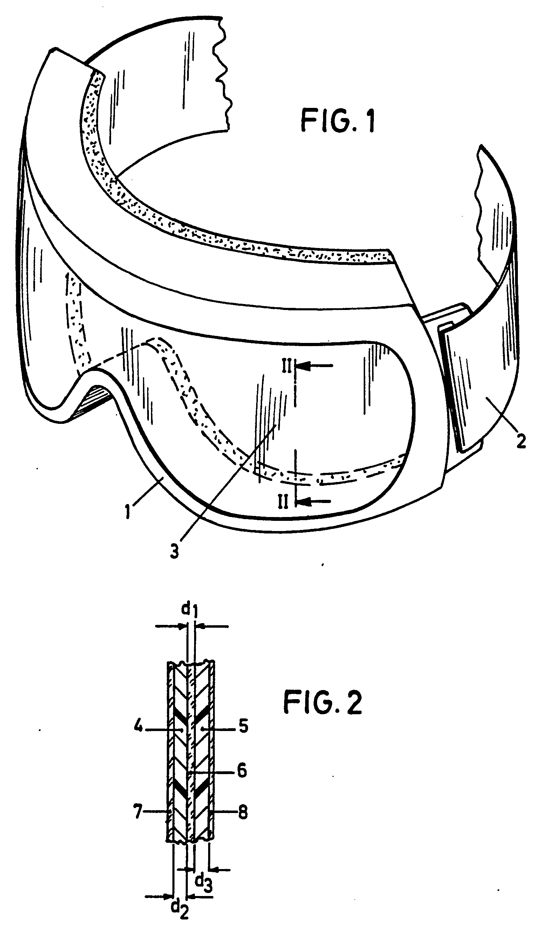

Eine in Fig. 1 dargestellte Skibrille umfaßt einen Rahmen 1 aus flexiblem Kunststoff, an dessen Außenseite ein Halteband 2 befestigt ist, und welcher eine aus einem Stück hergestellte transparente Scheibe 3 aufnimmt.

Die Scheibe 3 umfaßt gemäß der Schnittdarstellung in Fig. 2 eine transparente Kunststoffscheibe 4 aus Polycarbonat und eine transparente Scheibe 5 aus einem Zellulosederivat, im Ausführungsbeispiel Zelluloseacetat. Die beiden Scheiben 4, 5 sind flächig verklebt mittels einer Klebeschicht 6, welche durch einen Silikonkautschuk gebildet wird, wie er z.B. unter der Bezeichnung "Wacker RTV-2" im Handel ist. Die Dicke $d_1$ der Klebstoffschicht beträgt 70 μ, die Dicke $d_2$ bzw. $d_3$ der Polycarbonatscheibe 4 bzw. der Zelluloseacetatscheibe 5 beträgt jeweils 0,6 mm.

Die Scheibe 3 ist so in den Rahmen 1 eingesetzt, daß die Polycarbonatscheibe 4 nach außen und die Zelluloseacetatscheibe 5 innen zu liegen kommt.

Die Außenseite der Polycarbonatscheibe 4 ist in an sich bekannter Weise mit einem Lack 7 versehen, durch welchen die Oberflächenhärte und damit die Kratzfestigkeit wesentlich erhöht wird. Die Zelluloseacetatscheibe 5 ist an der dem Innenraum der Brille zugewandten Seite verseift, wodurch aufgrund dieses chemischen Vorgangs eine Schicht 8 entsteht, welche einem Beschlagen der Scheibe entgegenwirkt.

## Patentansprüche

1. Skibrille, Arbeitsschutzbrille, Motorradhelm-Visier oder dergleichen mit einer Scheibe umfassend zwei mittels eines Klebers verbundene transparente Kunststoffscheiben, wobei wenigstens die innere Scheibe 5 eine Cellulosederivatscheibe ist, welche mit der zweiten Scheibe 4 mittels eines Klebstoffes verklebt ist, dadurch gekennzeichnet, daß die beiden Scheiben mittels eines transparenten Klebstoffes (6) auf Silikonkautschuk-Basis flächig verklebt sind.

2. Skibrille, Arbeitsschutz-Brille, Motorradhelm-Visier o. dgl. nach Anspruch 1, dadurch gekennzeichnet, daß der Klebstoff (6) auf der Basis von Polydiacryl, Polydiaryl- bzw. Polyalkylarylsiloxanen mit reaktionsfähigen, insbesondere aciden Endgruppen hergestellt ist.

3. Skibrille Arbeitsschutz-Brille, Motorradhelm-Visier, od.dgl. nach Anspruch 1, dadurch gekennzeichnet, daß die zweite Scheibe (4) eine Polycarbonatscheibe ist.

4. Skibrille, Arbeitsschutz-Brille, Motorradhelm-Visier od.dgl. nach Anspruch 3, dadurch gekennzeichnet, daß die Polycarbonatscheibe (4) an ihrer Außenseite mit einer die Kratzfestigkeit erhöhenden Beschichtung (7) versehen ist.

5. Skibrille, Arbeitsschutz-Brille, Motorradhelm-Visier od.dgl. nach Anspruch 1, dadurch gekennzeichnet, daß die Zellulosederivat-Scheibe (5) an ihrer Außenseite mit einer Antibeschlag-Beschichtung (8) versehen, insbesondere verseift ist.

## Revendications

1. Lunettes de ski, lunettes de protection, visière de casque de motocyclette ou analogue, avec un verre composé de deux verres transparents en matière plastique, reliés entre-eux à l'aide d'une colle, le verre intérieur (5) au moins, se présentant sous la forme d'un verre en un dérivé de cellulose, qui est collé sur le deuxième verre (4) au moyen d'une colle, caractérisées en ce que les deux verres sont reliés entre-eux par un collage surfacique au moyen d'une colle transparente (6) à base de caoutchouc silicone.

2. Lunettes de ski, lunettes de protection, visière de casque de motocyclette ou analogue, selon la revendication 1, caractérisées en ce que la colle (6) est fabriquée à base de polydiacryl, polydiaryl- ou polyalkylarylsiloxanes, avec des groupes terminaux réactifs, notamment acides.

3. Lunettes de ski, lunettes de protection, visière de casque de motocyclette ou analogue, selon la revendication 1, caractérisées en ce que le deuxième verre (4) est un verre en polycarbonate.

4. Lunettes de ski, lunettes de protection, visière de casque de motocyclette ou analogue, selon la revendication 3, caractérisées en ce que le verre en polycarbonate (4) est pourvu sur son côté extérieur, d'un revêtement (7) augmentant la résistance aux rayures.

5. Lunettes de ski, lunettes de protection, visière de casque de motocyclette ou analogue, selon la revendication 1, caractérisées en ce que le verre (5) en un dérivé de cellulose est pourvu, sur son côté extérieur, d'un revêtement anti-buée (8), notamment par saponification.

## Claims

1. Skiing goggles, protective goggles, a motorcyclist's helmet visor or the like, with a sight piece comprising two transparent plastic pieces joined together by means of an adhesive, with at least the inner piece (5) being a cellulose derivative piece which is glued with the second piece (4) by means of an adhesive, characterised in that the two pieces are surface-glued by means of a silicone-based transparent adhesive (6).

2. Skiing goggles protective goggles a motorcyclist's helmet visor or the like, according to patent claim 1, characterised in that the adhesive (6) is made on a basis of polydiacryl, polydiaryl or polyalkylaryl siloxanes with reactive, particularly acid, end groups.

3. Skiing goggles, protective goggles, a motorcyclist's helmet visor or the like, according to patent claim 1, characterised in that the second piece (4) is a polycarbonate piece.

4. Skiing goggles, protective goggles, a motorcyclist's helmet visor or the like, according to patent claim 3, characterised in that the polycarbonate piece (4) is provided on its outer side with a coating (7) increasing resistance to scratching.

5. Skiing goggles, protective goggles, a motorcyclist's helmet visor or the like, according to patent claim 1, characterised in that the cellulose (derivative piece 5) is provided on its outer side with a coating preventing dimming or misting (8), and is in particular saponified.

FIG. 1

FIG. 2